# EUROPÄISCHE PATENTSCHRIFT

(11) **EP 1 341 924 B1**
(45) Veröffentlichungstag und Bekanntmachung des Hinweises auf die Patenterteilung: **06.05.2004**
(21) Anmeldenummer: 01270615.6
(22) Anmeldetag: 12.12.2001
(51) Int. Cl.: C12P 1/00, C12N 1/20

(54) **PRODUKTIONSVERFAHREN FÜR BIOMASSE**
METHOD FOR PRODUCING BIOMASS
PROCEDE DE PRODUCTION DE BIOMASSE

(30) Priorität: 13.12.2000 DE 10062030
(43) Veröffentlichungstag der Anmeldung: 10.09.2003
(73) Patentinhaber: HAMPP, Norbert, 35287 Amöneburg-Rossdorf (DE)
(72) Erfinder: HAMPP, Norbert, 35287 Amöneburg-Rossdorf (DE)
(74) Vertreter: Dey, Michael, Dr.
(86) Internationale Anmeldenummer: PCT/EP2001/014600
(87) Internationale Veröffentlichungsnummer: WO 2002/048381

(56) Entgegenhaltungen:
- DD-A- 269 163
- US-A- 4 826 769
- RODRIGUEZ-VALERA F.: "Biotechnological potential of halobacteria" BIOCHEM.SOC.SYMP., Bd. 58, 1992, Seiten 135-147, XP008002314

## Beschreibung

Die vorliegende Erfindung betrifft ein Verfahren zur Herstellung von Biomasse aus halophilen Organismen in großtechnischem Maßstab.

Die Herstellung von Biomasse in technischem Maßstab erfolgt üblicherweise in Fermentationsanlagen. Diese bestehen im Wesentlichen aus einem oder mehreren abgeschlossenen Gefäßen, die ein Volumen von typischerweise 10 bis 20 m³, im Einzelfall auch bis zu 100 m³ haben können. Weiterhin werden für technische Anlagen Hilfseinrichtungen zum Befüllen und Entleeren der Kultivierungsgefäße und zur Kontrolle der Kultivierungsbedingungen benötigt.

Für die Herstellung von größeren Mengen an Biomasse könnte auch eine Fermentation in offenen Becken in Betracht gezogen werden. Eine solche Verfahrensführung hat jedoch den Nachteil, dass ständig Kontaminationen von außen in das Kulturmedium eingetragen werden können. Weiterhin ist es bei einer Verfahrensführung in offenen Becken nicht auszuschließen, dass Fermentationsbrühe und die darin befindlichen Organismen in die Umwelt verschleppt und dort freigesetzt werden. Dies ist insbesondere dann nicht akzeptabel, wenn mit gentechnisch veränderten Organismen gearbeitet wird, die ggf. besonderen Sicherheitsauflagen unterliegen bzw. wenn mit anderen als gefährlich oder toxisch eingestuften Organismen gearbeitet werden soll.

Eine Aufgabe der Erfindung bestand deshalb darin, ein Herstellungsverfahren für Biomasse bereitzustellen, mit dem es möglich ist, große Volumina gegenüber dem Stand der Technik mit geringem Aufwand an Apparaturen und Kosten zu produzieren und gleichzeitig die Erfordernisse an für Mikroorganismen und insbesondere gentechnisch veränderte Mikroorganismen bestehenden Sicherheitsauflagen zu erfüllen.

Insbesondere bestand die Aufgabe darin, ein geeignetes Herstellungsverfahren für Biomasse aus halophilen Organismen bereitzustellen.

Diese Aufgabe wird erfindungsgemäß gelöst durch ein Verfahren zur Herstellung von Biomasse aus halophilen Organismen, welches dadurch gekennzeichnet ist, dass man eine Fermentation halophiler Organismen in einem Hohlraum in einem Salzstock durchführt und die halophilen Organismen oder Bestandteile davon als Biomasse gewinnt.

Der Begriff Fermentation bezeichnet allgemein Prozesse, bei denen unter aerobem oder anaerobem Stoffwechsel von Mikroorganismen, mit mikrobiellen Enzymen oder mit pflanzlichen oder tierischen Zellkulturen gewünschte Produkte gebildet werden. Eine Fermentation im erfindungsgemäßen Verfahren erfolgt unter Verwendung von halophilen Organismen.

Erfindungsgemäß können beispielsweise Stollen oder Kavernen in Salzstöcken bzw. Salzbergwerken als Reaktionsgefäße für eine Fermentation genutzt werden. Das Volumen von solchen Stollen oder Kavernen ist um Größenordnungen höher als das künstlicher Fermentationsgefäße. Auf diese Weise können beachtliche Mengen an Biomasse produziert werden ohne das Erfordernis, dass teuere Produktionsanlagen in großem Maßstab bereitgestellt werden müssten. Salzkavernen entstehen in Salzstöcken, Salzbergwerken oder Salzvorkommen üblicherweise durch Aussolung. Typische Dimensionen der bei einer Salzgewinnung durch Aussolung übrig bleibenden Salzkavernen sind beispielsweise Durchmesser von etwa 20 bis 200 m, insbesondere etwa 60 m, und Höhen von etwa 100 bis 500 m, insbesondere etwa 300 m. Solche Salzkavernen können ein Volumen von einigen Tausend Kubikmetern, beispielsweise von etwa 500 000 bis 800 000 m³ aufweisen und stellen somit riesige Reaktionsgefäße dar, welche erfindungsgemäß zur Herstellung von Biomasse genutzt werden.

Durch die günstigen Eigenschaften von Salz (insbesondere Steinsalz, NaCl) wird eine praktisch vollständige Abdichtung gegenüber der Umgebung erzielt. Dadurch sind die Salzkavernen in idealer Weise dicht und stabil, um als Fermentationsreaktionsraum zu dienen. Weiterhin lassen sich die unterirdisch liegenden Stollen und Kavernen vollständig von der Außenwelt abschließen, so dass eine Produktion auch unter Beachtung von strengsten Sicherheitsauflagen durchgeführt werden kann. Eine Kontamination der Umwelt oder durch die Umwelt, wie sie ein großes Problem bei offenen Becken und sonstigen Reaktionsgefäßen darstellt, ist bei der erfindungsgemäßen Verfahrensweise ausgeschlossen.

Aufgelassene Salzbergwerkskavernen und -stollen gibt es weltweit in großen Mengen, so dass hier leicht verfügbare und günstige Reaktionsgefäße zur Verfügung stehen. Bisher werden solche Salzkavernen beispielsweise als Lagerstätten für Abfälle, insbesondere radioaktive Abfälle, und zur Einlagerung von Erdöl oder Erdgas genutzt. Diese herkömmliche Nutzungsart von Salzkavernen wird beispielsweise unter http://www.kbbnet.de beschrieben.

Ein weiterer Anwendungsbereich aus dem Stand der Technik ist die Nutzung von unterirdischen Hohlräumen, wie z.B. Salz-Kavernen, zum gezielten Abbau von organischem Material (z.B. Kohle) zu z.B. Methan mit Hilfe von Mikroorganismen.

US-Patente Nr. 4,826,769 und 4,845,034 beschreiben den Abbau von Kohle durch eine biochemische Reaktion zu Produkten wie z.B. Methan durch anaerobe Mikroorganismen in unterirdischen Hohlräumen, wie z.B. unterirdischen Salz-Kavernen.

US-Patent Nr. 6,143,534 bezieht sich auf die Gewinnung von gasförmigen Brennstoffgasen aus Lignin-Substraten (wie z.B. Kohle) durch eine biochemische Reaktion, wofür geeignete anaerobe Mikroorganismen eingesetzt werden. Bei der Nutzung unterirdischer Salz-Kavernen als Reaktionsgefäß gemäß US-Patent Nr. 6,143,534, werden halophile Mikroben unter anaeroben Bedingungen bei der Biogasifikationsreaktion zur Bildung von insbesondere Methan eingesetzt.

Im Gegensatz zu diesem Stand der Technik werden bei der vorliegenden Erfindung Hohlräume in Salzstöcken nicht dazu verwendet, Mikroorganismen für den Zweck zu kultivieren, organisches Material abzubauen bzw. umzuwandeln, sondern es wird der Aufbau von Biomasse beschrieben. Gemäß der Erfindung werden halophile Organismen kultiviert und die halophilen Organismen oder Bestandteile davon als Biomasse gewonnen.

Zahlreiche Fermentationen müssen, bedingt durch die verwendeten Organismen, unter anaeroben Bedingungen durchgeführt werden. Da sich die erfindungsgemäß genutzten Hohlräume, beispielsweise Kavernen, leicht luftdicht abschließen lassen, können auf einfache Weise anaerobe Bedingungen im Kulturmedium eingestellt werden. Beim erfindungsgemäßen Verfahren wird deshalb in einer bevorzugten Ausführungsform die Fermentation unter anaeroben Bedingungen durchgeführt. Vor allem vorteilhaft bei der erfindungsgemäßen Verfahrensführung ist, dass der Sauerstoffanteil der Atmosphäre, in der das Verfahren durchgeführt wird, reguliert werden kann. Das bedeutet, dass man anaerob, partiell anaerob, aerob oder auch abwechselnd anaerob und aerob arbeiten kann.

In sauerstoffreicher (aerober) Atmosphäre werden halophile Organismen im Allgemeinen vermehrt. Die Bildung von Biomassebestandteilen, wie z.B. der Purpurmembran, wird hingegen in sauerstoffarmer bzw. anaerober Atmosphäre induziert. Bevorzugt wird bei dem erfindungsgemäßen Verfahren die Vermehrung der halophilen Organismen unter aeroben und die Induktion der Bildung von Biomassebestandteilen, wie z.B. der Purpurmembran, unter anaeroben Bedingungen durchgeführt.

Möglich ist auch, das Verfahren vollständig im partiell anaeroben Bereich durchzuführen, sodass Vermehrung und Induktion parallel ablaufen. Wenn lediglich die Vermehrung der halophilen Organismen erwünscht ist, kann das Verfahren durchgehend aerob geführt werden. Dies ist beispielsweise dann von Nutzen, wenn letztendlich nicht z.B. die Purpurmembran sondern andere Bestandteile der Biomasse, wie z.B. DNA und Proteine, gewonnen werden sollen.

Bei anaerober Verfahrensführung wird das Verfahren sauerstofffrei oder mit geringen Mengen von bis < 3 Vol.-%, bevorzugt ≤ 2,5 Vol.-%, mehr bevorzugt ≤ 2 Vol.-% und besonders bevorzugt ≤ 1,5 Vol.-% Sauerstoff durchgeführt. Anaerob bedeutet insbesondere vollkommen sauerstofffrei (= 0 % Sauerstoffgehalt) oder in Gegenwart einer geringen O₂-Menge von z.B. ≥ 0,5, ≥ 1 Vol.-%.

Partiell anaerobe Durchführung des Verfahrens bedeutet, dass bei Sauerstoffgehalten von 3 bis 8 Vol.-% gearbeitet wird. Bevorzugt wird ein O₂-Gehalt von 3,5 bis 5 Vol.-% eingestellt.

Der O₂-Gehalt bei aerober Verfahrensführung beträgt üblicherweise zwischen > 8 Vol.-% und dem O₂-Gehalt der Luft, also ca. 21 Vol.-%, bevorzugt ≥ 10 Vol.-%, mehr bevorzugt ≥ 15 Vol.-%. Die Angaben des O₂-Gehaltes beziehen sich dabei auf den O₂-Anteil in der dem Reaktionsmedium zugeführten Gasmischung bzw. oberhalb des Reaktionsmediums. Die Messung des O₂-Gehaltes im Gasraum kann mit konventionellen Methoden, wie z.B. mittels Sauerstoff-Elektroden erfolgen.

Der O₂-Gehalt wird entsprechend der gewünschen Verfahrensführung eingestellt. Dies kann auf einfache Weise z.B. über Kunststoffschläuche mit kontrollierter Porösität erreicht werden.

Die Menge des in der Reaktionslösung gelösten Sauerstoffs hängt von der zugeführten O₂-Menge bzw. von der im Gasraum vorhandenen O₂-Menge ab, wobei sich ein Gleichgewicht zwischen dem in der Salzlösung gelösten und dem im Gasraum vorliegenden Sauerstoff einstellt. Bei der Durchführung des erfindungsgemäßen Verfahrens wird Sauerstoff in das Reaktionsmedium bzw. in den Gasraum geleitet, der sich dann entsprechend in der Salzlösung löst. Unter diesen dann vorliegenden aeroben Bedingungen vermehren sich die Organismen und verbrauchen dabei Sauerstoff, wodurch sich, sobald kein weiterer Sauerstoff zugeführt wird, ein anaerobes Medium ausbildet. Je nachdem, ob eine weitere Vermehrung erwünscht ist, kann erneut begast werden. Wenn die Induktion der Bildung von z.B. der Purpurmembran erfolgen soll, werden die anaeroben Bedingungen aufrechterhalten. Durch entsprechend häufig erfolgende Begasung kann auch vollständig aerob gearbeitet werden, ohne Begasung kann eine vollständig anaerobe Verfahrensführung erreicht werden. Um das Verfahren durchgehend im partiell anaeroben Bereich zu halten, kann durch Begasung in Intervallen ein konstantes O₂-Level gehalten werden.

Die Löslichkeit von Sauerstoff in der wässrigen Salzlösung ist Temperaturabhängig. Die üblichen Fermentationstemperaturen sind vorzugsweise 10 bis 80 °C, mehr bevorzugt 25 bis 45 °C, am meisten bevorzugt 30 bis 40 °C. Um die erforderlichen Temperaturen einzustellen, kann es notwendig sein, das Medium zu beheizen. Dies kann über konventionelle Methoden oder auch gegebenenfalls über Sonneneinstrahlung erfolgen.

Beim erfindungsgemäßen Verfahren zur Herstellung von Biomasse werden halophile, bevorzugt halotolerante oder/und extrem halophile Organismen kultiviert, besonders bevorzugt Halobakterien. Bevorzugt werden Halobacterium salinarum oder/und Haloferax volcanii kultiviert. Die Kultivierung solcher Organismen erfolgt in salzhaltigen Medien, wobei der Salzgehalt auf den betreffenden zu kultivierenden Organismus eingestellt wird. Im Allgemeinen sollte das Medium einen Salzgehalt von etwa 5 Gew.-% bis zu dem Gehalt einer gesättigen Lösung, z.B. bis etwa 40 Gew.-%, haben. Bei Raumtemperatur enthält eine gesättigte Salzlösung etwa 26 Gew.-% Salz. Eine Salzlösung mit etwa 5 Gew.-% Salz entspricht in etwa der Meerwassersalzkonzentration.

Halophile ("Salz-liebende") Organismen sind Organismen, die in Medien mit etwa 5 Gew.-% Salzanteil, d.h. in einer ca. 1 M Salzlösung, optimal wachsen, also z.B. in Meerwasser kultivierbar sind. Lebensfähig sind solche Organismen bei Salzkonzentrationen von mindestens etwa 3 Gew.-%.

Die optimale Vermehrung extrem halophiler Organismen bzw. halotoleranter Organismen verläuft bei Salzkonzentrationen > 2,5M (ca. > 15 Gew.-%ige Salzlösung). Beispiele extrem halophiler Organismen sind Haloferax volcanii und Halobacterium salinarum. Extrem halophile Organismen sind bei Konzentrationen von etwa ≥ 10 Gew.-% lebensfähig.

Halotolerante Organismen können in extrem salzhaltiger Umgebung wachsen (> 2,5 M), haben aber den hohen Salzgehalt nicht in der Zelle, sondern kompensieren den osmotischen Druck durch die Produktion eines geeigneten Stoffes. Dunaniella ist beispielsweise eine halotolerante Spezies, die Glycerin produziert. Diese Organismen sind sowohl bei geringen als auch bei sehr hohen Salzkonzentrationen (gesättigte Lösung) lebensfähig, also z.B. bei bis etwa 26 Gew.-% Salz.

Die Fermentation von halophilen und insbesondere von extrem halophilen oder/und halotoleranten Organismen hat bisher bei den im Stand der Technik bekannten Fermentationsanlagen erhebliche zusätzliche kostenintensive apparative Anforderungen impliziert, da Kulturgefäße mit hoher Korrosionsbeständigkeit, beispielsweise Kulturgefäße mit Keramikauskleidung, verwendet werden müssen. Bei der erfindungsgemäßen Verfahrensführung in einem Hohlraum in einem Salzstock entfallen diese zusätzlichen apparativen Anforderungen. Da als Reaktionsgefäß eine Kaverne in einem Salzstock eingesetzt wird, stehen zudem die zur Kultivierung von halophilen Organismen benötigten sehr großen Salzmengen unmittelbar zur Verfügung. Beispielsweise kann das Salz von der Wand der Kaverne einfach mit Wasser in die Fermentationsbrühe gespült werden, wodurch die für die Kultivierung von halophilen Organismen notwendigen hohen Salzkonzentrationen automatisch gegeben sind. Durch dieses einfache System ist es möglich, eine konstante Zelldichte zu halten, was besonders gute Ausbeuten ergibt. Durch einfaches Zudosieren bzw. Entfernen von Wasser lässt sich das Volumen der Fermentationslösung der gewünschten Zelldichte anpassen, wobei sich automatisch wieder die gewünschte konzentrierte Salzlösung einstellt, da bei Entfernen von Wasser Salz ausfällt und sich bei Hinzufügen von Wasser eine adäquate Menge Salz von der Wandung bzw. aus dem Bodenvorrat löst. Zur Durchführung einer Fermentation halophiler Organismen wird in dem Hohlraum in einem Salzstock vorzugsweise eine gesättigte Salzlösung verwendet, die einen Salzanteil von vorzugsweise ca. 26 Gew.-% bei RT aufweist. Ein zusätzlicher Vorteil der Durchführung von Fermentationen in gesättigten Salzlösungen besteht darin, dass eine Kontamination durch andere störende Mikroorganismen bei derart hohen Salzkonzentrationen praktisch nicht auftritt. Wird für die Kultivierung der Organismen eine Salzkonzentration benötigt, die geringer ist als eine gesättigte Lösung, so kann die entsprechende Konzentration der Lösung eingestellt werden, indem kontinuierlich mit einer bestimmten Zulaufgeschwindigkeit Wasser zugegeben wird, sodass sich das Salz langsamer aus der Wandung bzw. dem Gefäßboden löst als die Wasserzugabe erfolgt. Auf diese Weise ist es möglich, Organismen zu kultivieren, deren optimales Wachstum in konzentrierten aber nicht gesättigten Salzlösungen erfolgt, wobei die Zelldichte gleichzeitig auch hier reguliert bzw. konstant gehalten werden kann.

Weiterhin entfallen Transport- und Lagerungskosten, da das zur Kultivierung benötigte Salz unmittelbar an der Produktionsstätte vorhanden ist.

Eine besonders interessante Klasse extrem halophiler Organismen sind Halobakterien-Stämme, die Bakteriorhodopsin, Purpurmembran, weiße Membran, Halorhodopsin, Sensorrhodopsin I oder/und II, ATPasen, Flagellarmotoren und andere technisch nutzbare Enzyme oder Produkte herstellen. Erfindungsgemäß können als Fermentationsprodukte auch Substanzen hergestellt werden, die durch Modifikation, insbesondere durch gentechnologische Modifikation der genannten Produkte erhalten werden, sowie Produkte, die erst nach einer gentechnologischen Modifikation der Mikroorganismen von diesen produziert werden. Erfindungsgemäß können somit alle bekannten Produkte von Halobakterien sowie diese Produkte in modifizierter Form als Biomasse erhalten werden. Darüber hinaus ist es möglich, auch nicht-natürliche Produkte in Mikroorganismen, insbesondere in Halobakterien herzustellen, also Produkte, welche die Mikroorganismen erzeugen, nachdem ihnen die entsprechende genetische Information eingepflanzt worden ist. In diesem Fall fungieren die Mikroorganismen, insbesondere Halobakterien als reiner Wirt.

Um die gewünschten Produkte, wie etwa Bakteriorhodopsin zu erhalten, ist es bei der Kultivierung von Halobakterienstämmen jedoch häufig erforderlich, das Kulturmedium zusätzlich mit Licht zu beleuchten oder eine anaerobe Kultivierung durchzuführen. Da, wie oben erläutert, beim erfindungsgemäßen Verfahren eine anaerobe Verfahrensführung auf einfache Weise möglich ist, kann auf die im Stand der Technik benötigten Einbauten, die eine Beleuchtung bzw. Begasung bzw. Evakuierung ermöglichen, verzichtet werden.

Da darüber hinaus die für die Fermentation von halophilen Organismen benötigten Salzmengen direkt aus dem Salzvorkommen bzw. dem Salzstock entnommen werden können, ist das erfindungsgemäße Verfahren sowohl in apparatetechnischer, logistischer, als auch kostentechnischer Sicht deutlich günstiger als herkömmliche Verfahren.

Das erfindungsgemäße Verfahren kann auch eingesetzt werden, um gentechnisch veränderte Organismen zu kultivieren. Auf diese Weise können spezifische, gewünschte, ggf. modifizierte Fermentationsprodukte erhalten werden. Die Verarbeitung bzw. Prozessierung von gentechnisch veränderten Organismen ist möglich, da die Kavernen leicht vollständig von der Außenwelt abgeschlossen werden können und somit neben einem Luftausschluss auch eine Kontamination in bzw. aus dem Kultivierungsmedium praktisch vollständig vermieden werden kann.

Somit kann mit dem erfindungsgemäßen Verfahren der gewünschte Bestandteil halophiler Organismen, beispielsweise Bakteriorhodopsin oder ein gentechnisch modifiziertes Bakteriorhodopsin, die Purpurmembran, die weiße Membran oder ein anderes in Zellkulturen erzeugtes Produkt in großen Mengen als Fermentationsprodukt gewonnen werden. Beispielsweise kann pro Kaverne Biomasse in einer Menge von etwa 50 Tonnen/Jahr gewonnen werden. Die Biomasse kann aus dem Medium kontinuierlich oder chargenweise entnommen werden.

Bei entsprechender Verfahrensführung enthalten die kultivierten halophilen Organismen weitere Bestandteilen wie z.B. DNA und lösliche Proteinen, die gegebenenfalls als Biomasse gewonnen werden können. DNA oder/und lösliche Proteine können z.B. teilweise oder vollständig unmittelbar vor Ort (sauer bzw. alkalisch oder mechanisch oder thermisch) aufgeschlossen werden und dann so als Kohlenstoffquellen für weitere Fermentationen verwendet oder auch als Biomasse isoliert werden.

Bei den verwendeten Hohlräumen kann es sich sowohl um einen natürlichen als auch um einen künstlich gebildeten Hohlraum in einem Salzstock bzw. Salzvorkommen handeln. Künstlich hergestellte Hohlräume entstehen insbesondere bei der Aussolung von Salzformationen zur Gewinnung von Salz. Die Hohlräume können beliebige Volumina aufweisen, wobei Volumina in der Größenordnung von einigen Hunderttausend m³ für Salzkavernen üblich sind.

Durch die erfindungsgemäße Verwendung von Hohlräumen in Salzstöcken als Reaktionsgefäße bei der Herstellung von Biomasse bzw. bei der Herstellung von gewünschten Fermentationsprodukten in großen Mengen werden insbesondere folgende Vorteile erhalten. Zunächst sind die Kosten pro Fermentationsvolumen wesentlich günstiger als bei üblicherweise verwendeten Fermentationsanlagen, da Salzkavernen bereits in großer Zahl existieren und meist ungenutzt sind. Weiterhin können die enormen Salzmengen, die für die Fermentation für extrem halophilen Organismen benötigt werden, kostengünstig unmittelbar aus dem Salzstock entnommen werden, so dass auch keine Transport- oder Lagerungsprobleme entstehen. Weiterhin können Kavernen in Salzstöcken leicht vollständig gegenüber der Außenwelt abgeschlossen werden. Dadurch wird zum einen eine Kontamination der Fermentationsbrühe durch Verunreinigungen von außen ausgeschlossen, zum anderen wird verhindert, dass Bestandteile der Fermentationsbrühe, beispielsweise Organismen und insbesondere gentechnisch veränderte Organismen, in die Außenwelt gelangen können. Dadurch kann eine Produktion unter Verwendung von gentechnisch veränderten Organismen selbst unter strengen biologischen Sicherheitsauflagen etabliert werden. Durch den Abschluss gegenüber der Außenwelt ist es weiterhin möglich, die Reaktion gleich unter anaeroben Bedingungen durchzuführen.

Da Salzkavernen vollständig von dicken Lagen an Salz umgeben sind, besteht keinerlei Verbindung zum Grundwasser. Eine Kontamination des Grundwassers aus dem Fermentationsmedium heraus mit Inhaltsstoffen bzw. Mikroorganismen ist deshalb ausgeschlossen. Dies stellt auch einen Vorteil von Hohlräumen in Salzstöcken gegenüber normalen Höhlen dar, da. Höhlen in der Regel immer irgendwelche Spalten etc. aufweisen.

Ein weiterer technischer Vorteil des erfindungsgemäßen Verfahrens ist, dass das Fermentationsmedium nach der Fermentation und der Abtrennung der Mikroorganismen direkt als Flüssigsalz für z.B. die Enteisung von Straßen eingesetzt werden kann. Da Halobakterien z.B. unter Wasserzusatz lysieren, würden sie bei Ausbringen in die Umwelt und der resultierenden Verdünnung mit Wasser automatisch lysieren, sodass eine solche Weiterverwendung der Salzlösung unbedenklich ist.

Die Fermentation selbst wird bevorzugt in einem unterirdischen künstlichen Salzsee ausgeführt, der durch Einsprühen, Einspritzen oder Einspülen von Wasser in den Hohlraum gebildet wird. Bevorzugt wird beim Anlegen des künstlichen Salzsees Salz von der Wand der Kaverne mit Wasser abgespült, so dass das gewünschte Nährmedium unmittelbar entsteht. Dabei können die erforderlichen Salzkonzentrationen eingestellt werden. Auf diese Weise kann das zur Kultivierung von halophilen Bakterien benötigte Salz einfach zugeführt werden, ohne dass hierzu technisch aufwendige Apparaturen notwendig wären.

### Beispiel

Das erfindungsgemäße Verfahren wird bevorzugt wie folgt durchgeführt:
1.1 Herstellung der Fermentationsbrühe
   Durch Abspülen von Salz von den Wänden der Kaverne mit Wasser wird zunächst eine Salzlösung gebildet. Anschließend kann die Ionenzusammensetzung der Salzlösung analysiert werden und die Lösung ggf. durch Zusatz weiterer Ionen, z.B. Kalium, Magnesium, Calcium, etc. in Mengen, wie sie für die Fermentation benötigt werden, ergänzt werden. Der gebildete Salzsee kann ggf. thermostatisiert werden, indem die Sole, je nach Größe der Kaverne, gerührt oder umgepumpt wird. Bevorzugt zieht man mit einem Rohr Sole vom Grund des Sees nach oben, thermostatisiert diese und führt sie dem See oben wieder zu. Durch eine Thermostatisierung kann das Wachstum der Mikroorganismen und damit die Menge des Fermentationsproduktes weiter erhöht werden. Schließlich können der Salzlösung noch Kohlenstoff-Quellen für die Fermentation zugesetzt werden, beispielsweise Pepton oder Stärkehydrolysate.
1.2 Durchführung der Fermentation
   Der wie oben hergestellte Salzsee, der als Fermentationsbrühe dient, wird mit einer Bakterienkultur, insbesondere einer Halobakterienkultur angeimpft. Anschließend wird das Wachstum der Bakterien verfolgt, bis eine akzeptable oder vorbestimmte Zelldichte erreicht ist. Anschließend erfolgt die Abtrennung der gebildeten Biomasse aus dem Medium. Die Abtrennung kann kontinuierlich erfolgen, z.B. durch die sog. Querstromfiltration (Cross-flow Module). Es ist aber auch möglich, die Entnahme im Batch-Verfahren durchzuführen. Die zusammen mit der Biomasse entfernte Salzmenge wird durch die temperaturabhängige Sättigungslöslichkeit von Salz in Wasser bzw. der Sole kontinuierlich von den Wänden nachgeliefert, indem es sich einfach auflöst.

## Patentansprüche

1. Verfahren zur Herstellung von Biomasse aus halophilen Organismen, **dadurch gekennzeichnet, dass** man eine Fermentation halophiler Organismen in einem Hohlraum in einem Salzstock durchführt und aus den halophilen Organismen Bakteriorhodopsin, Purpurmembran oder/und weiße Membran als Biomasse gewinnt.

2. Verfahren nach Anspruch 1, **dadurch gekennzeichnet, dass** das Verfahren zunächst aerob zur Vermehrung der halophilen Organismen und dann anaerob zur Induktion der Bildung von Biomasse-Bestandteiten durchgeführt wird.

3. Verfahren nach Anspruch 1, **dadurch gekennzeichnet, dass** das Verfahren partiell anaerob durchgeführt wird.

4. Verfahren nach einem der vorhergehenden Ansprüche **dadurch gekennzeichnet, dass** man halotolerante oder/und extrem halophile Organismen einsetzt.

5. Verfahren nach einem der vorhergehenden Ansprüche, **dadurch gekennzeichnet, dass** man Halobakterien einsetzt.

6. Verfahren nach einem der vorhergehenden Ansprüche, **dadurch gekennzeichnet, dass** man *Haloferax volcanii*, oder/und *Halobacterium salinarum* einsetzt.

7. Verfahren nach einem der vorhergehenden Ansprüche, **dadurch gekennzeichnet, dass** man einen natürlichen oder/und künstlich gebildeten Hohlraum nutzt.

8. Verfahren nach einem der vorhergehenden Ansprüche, **dadurch gekennzeichnet, dass** die für die Fermentation von halophilen Organismen benötigten Salzmengen aus dem Salzstock entnommen werden.

9. Verfahren nach einem der vorhergehenden Ansprüche, **dadurch gekennzeichnet, dass** gentechnisch veränderte Organismen eingesetzt werden.

## Claims

1. Method for producing biomass from halophilic organisms, **characterized in that** halophilic organisms are fermented in a hollow space in a salt dome and bacteriorhodopsin, purple membrane or/and white membrane are isolated as biomass from the halophilic organisms.

2. Method as claimed in claim 1, **characterized in that** the method is firstly carried out aerobically to multiply the halophilic organisms and then anaerobically to induce the formation of biomass components.

3. Method as claimed in claim 1, **characterized in that** the method is carried out partially anaerobically.

4. Method as claimed in one of the previous claims, **characterized in that** halotolerant or/and extremely halophilic organisms are used.

5. Method as claimed in one of the previous claims, **characterized in that** halobacteria are used.

6. Method as claimed in one of the previous claims, **characterized in that** *Haloferax volcanii* or/and *Halohacterium salinarum* are used.

7. Method as claimed in one of the previous claims, **characterized in that** a natural hollow space or/and one that is formed artificially is used.

8. Method as claimed in one of the previous claims, **characterized in that** the amounts of salt required for the fermentation of halophilic organisms are removed from the salt dome.

9. Method as claimed in one of the previous claims, **characterized in that** genetically modified organisms are used.

## Revendications

1. Procédé de fabrication de biomasse à partir d'organismes halophiles, **caractérisé en ce qu'**on réalise une fermentation d'organismes halophiles dans une cavité d'un gisement de sel et, à partir des organismes halophiles, on obtient une bactériorhodopsine, une membrane pourpre ou/et une membrane blanche en tant que biomasse.

2. Procédé selon la revendication 1, **caractérisé en ce qu'**on réalise le procédé d'abord de manière aérobie pour multiplier les organismes halophiles, puis de manière anaérobie pour induire la formation de constituants de biomasse.

3. Procédé selon la revendication 1, **caractérisé en ce qu'**on réalise le procédé de manière partiellement anaérobie.

4. Procédé selon l'une quelconque des revendications précédentes, **caractérisé en ce qu'**on met en oeuvre des organismes halotolérants ou/et extrêmement halophiles.

5. Procédé selon l'une quelconque des revendications précédentes, **caractérisé en ce qu'**on met en oeuvre des halobactéries.

6. Procédé selon l'une quelconque des revendications précédentes, **caractérisé en ce qu'**on met en oeuvre *Haloferax volcanii*, oulet *Halobacterium salinarum*.

7. Procédé selon l'une quelconque des revendications précédentes, **caractérisé en ce qu'**on utilise une cavité naturelle ou/et artificielle.

8. Procédé selon l'une quelconque des revendications précédentes, **caractérisé en ce que** les quantités de sel nécessaires à la fermentation d'organismes halophiles sont prélevées dans le gisement de sel.

9. Procédé selon l'une quelconque des revendications précédentes, **caractérisé en ce qu'**on met en oeuvre des organismes génétiquement modifiés.
